Europäisches Patentamt

European Patent Office

Office européen des brevets

(11) Publication number: **0 371 137**
**A1**

(12) **EUROPEAN PATENT APPLICATION**
published in accordance with Art.
158(3) EPC

(21) Application number: 89902613.2

(51) Int. Cl.⁵: **A61K 43/00**

(22) Date of filing: 04.11.88

(86) International application number:
**PCT/SU88/00224**

(87) International publication number:
**WO 89/09623 (19.10.89 89/25)**

(30) Priority: 15.04.88 SU 4411966

(43) Date of publication of application:
**06.06.90 Bulletin 90/23**

(84) Designated Contracting States:
**AT BE CH DE FR GB IT LI LU NL SE**

(71) Applicant: **ORENBURGSKY
GOSUDARSTVENNY MEDITSINSKY INSTITUT
ul. Sovetskaya 6
Orenburg, 460834(SU)**

(72) Inventor: **NIKITENKO, Vyacheslav Ivanovich
ul. Volodarskogo, 27-53
Orenburg, 460000(SU)**

(74) Representative: **Lord, Hilton David et al
Marks & Clerk 57-60 Lincoln's Inn Fields
London WC2A 3LS(GB)**

(54) **PREPARATION FOR TRANSPORTING RADIONUCLIDES IN A LIVING ORGANISM.**

(57) An agent for transportation of radionuclides in a live organism in the form of cells of at least one non-pathogenic microorganism of bacteria capable of penetrating from the gastro-intestinal tract into tissues of the spleen, lympth nodes, lymphoid follicles of the intestine and into an inflammatory focus and of concentrating therein, and containing 10 to $2 \times 10^9$ live cells of the microorganisms in 1 mg of the agent.

## AGENT FOR TRANSPORTATION OF RADIONUCLIDES
## IN A LIVE ORGANISM

### Field of the Invention

The present invention relates to medicine and, more particularly, to a novel agent for transportation of radionuclides in a live organism.

### Prior Art

Radionuclides are widely employed for diagnosis and treatment of pathological processes in medical practice ("Klinicheskaya Rentgenoradiologiya", 1985, Moscow, Meditsyna Publishing House, vol. 4, pp. 3-21, 255-261). However, this is associated with a number of problems of their accumulation only in strictly specified regions or sites of tissues. Known in the art is but a limited number of tissue-tropic radionuclides. In view of this, widely used are pharmaceutical preparations which are carriers for radionuclides. For this purpose use is made of erythrocytes, plasma, aminoacids and other preparations. However, at the present time there is lack of sufficiently effective preparations of the radiopharmaceutical application for investigation of inflammatory processes, lympth nodes, spleen, lymphoid follicles of the intestine.

Furthermore, owing to a small radiation load, undesirable side effects (toxicity, allergization and the like) the known radiopharmaceutical preparations are not employed for therapeutic purposes.

### Disclosure of the Invention

The agent for transportation of radionuclides in a live organism according to the present invention is novel and hitherto unknown in the literature.

The present invention is directed to the provision of a novel agent ensuring transportation of radionuclides from the gastro-intestinal tract to the inflammatory focus, spleen, lymph nodes, lymphoid follicles of
the intestine, for diagnostic and therapeutic purposes,
which would be not toxic and possessing no side effects.

This problem is solved by that the agent for transportation of radionuclides according to the present invention in a live organism comprises cells of at least
one non-pathogenic microorganism of bacteria capable
of penetrating from gastro-intestinal tract into tissues
of the spleen, lympth nodes, lymphoid follicles of the
intestine and into the inflammatory focus and of being
concentrated therein; it contains 10 to $2 \times 10^9$ live
cells of the microorganism in 1 mg of the agent.

The agent according to the present invention pre-
ferrably contains, as bacteria, the following strains
of microorganisms:

- strain Bacillus subtilis  534 deposited on
March 28, 1988 at the All-Union Collection of microorganisms of the Institute of biochemistry and physiology
of microorganisms, the USSR Academy of Sciences, registered under No. B-1666D;

- strain Bacillus sp. 538 deposited on June 20,
1988 at the All-Union Collection of microorganisms of
the All-Union Research Institute of genetics and selection of industrial microorganisms registered under
No. BKPM-4401;

- strain Bacillus pulvifaciens deposited on April 14,
1988   at   the All-Union Collection of industrial
microorganisms of the All-Union Research Institute of
genetics and selection of industrial microorganisms
and registered under No. BKPM B-4348;

- strain Escherichia coli M17;

- strain Lactobacillus plantarus 8-FA-3;

- strain Bacteriumbifidum bifidum N 1.

- 3 -

The new agent according to the present invention makes it possible to transport the active principle to the tissues of the internal medium by an optimal way - - through the gastro-intestinal tract. Depending on the strain employed and the administered dose it is concentrated within 2 to 24 hours in the inflammatory focus, tissues of the spleen, lymph nodes, and to a lesser extent, in lymphoid follicles of the intestine and liver. This makes it possible to radically lower the dose of the active principle introduced into the organism and, hence, to limit the undesirable effect on other healthy organs and tissues. Within 24 - 72 hours 50 to 80% of the preparation are withdrawn from the organism mainly via the liver and the gastro-intestinal tract. The agent according to the present invention can remain in the tissues for a period of up to 14 days. This enables its use as an agent for transportation of pharmaceutical preparations for therapeutic purposes. Moreover, this agent based on the strains according to the present invention also produces a therapeutic effect _per se_. In tissues of the inner medium of the organism the agent inhibits growth of gram-positive and gram-negative bacteria, fungi, contributes to purification from necrotized tissues, arrests allergic responses.

Best Mode for Carrying out the Invention

The agent according to the present invention is prepared from non-pathogenic microorganisms of bacteria capable of penetrating from the gastro-intestinal tract into the spleen, lymph nodes, lymphoid follicles of the intestine, into the inflammatory focus and of being concentrated therein.

Microorganisms are isolated from human and animal organisms, first of all, from wounds healing by the first intention. In experiments those out of them are selected that can penetrate into the inflammatory focus, tissues of spleen, lympth nodes, lymphoid follicles of the intestine. The thus-obtained strains are tested for

pathogenieity, virulence, acute and chronical toxicity, allergenic effects. In the case of absence of undesirable side effects the strains are used as a new transportation agent. To this end, the strains are cultivated on nutrient media and lyophilized at a temperature drop of from -40 to +40°C under a pressure of from 5 to 70 Pa for 24 hours. The resulting powder-like preparation is stored in sealed ampules at room temperature. When required, radionuclides are incorporated into the matrix of the agent of the present invention. To this end, the lyophilized culture is inoculated and grown for 12 to 200 hours on media containing radionuclides. It is possible to incorporate radionuclides into the agent according to the present invention by way of contacting same with a suspension of live bacteria in aqueous solutions of salts or sugars at a temperature of 36 - 37°C for 0.1 - - 12 hours. Thereafter, the bacteria are separated from the medium, dried or a suspension in aqueous solutions of salts or sugars is prepared from them. The number of live bacteria in the ready agent can be varied from 10 to $2 \times 10^9$ cells in 1 mg of the agent. The specific activity of radionuclides can be varied from 0.01 to 10 MBq in 1 mg of the agent of the present invention or from 0.01 to 50 MBq (megaBecquerel) in 1 $cm^3$ of the suspension. The agent according to the present invention is administeried per os.

For the preparation of the agent according to the present invention use is preferably made of the following novel strains:

strain <u>Bacillus subtilis</u> 534, strain <u>Bacillus sp.</u> 538, strain <u>Bacillus pulvifaciens</u> 535.

It is also advisable to use known strains such as <u>Escherichia coli</u> M17, <u>Lactobacillus plantarum</u> 8-PA-3 and <u>Bacterium bifidum bifidum</u> I.

The novel strain <u>Bacillus subtilis</u> 534 is isolated from a non-purulent wound of a patient and has the following morphological features and physiological properties.

These are bacilli. The size of cells of a one-day agar culture is (2 - 4) x (0.6 - 0.8) µm. Bacteria virulent. Form spores, do not form capsules. Gram-positive. Colonies on a meat-peptone agar are rough with scalloped edges, slight pink shade, 2 - 12 mm diameter. The strain is multiplied at a temperature within the range of from 15 to 50°C, optimal growth temperature 36 - 37°C. Cleaves glucose, saccharose, mannitol to an acid without evolution of a gas. Does not ferment lactose. Does not form hydrogen sulphide and indole. Sensitive to benzylpenicillin, ampicillin, erythromycin, monomycin, linkomycin, tetracycline, insensitive to polymixin.

The strain produces an antibiotic with a broad range of action inhibiting growth of staphylococci, streptoccoci, proteus, blue pus bacillus, yeast fungi and the like. Furthermore, it releases proteolytic enzymes cleaving proteins, and an immunomodulator.

The novel strain Bacillus sp. 538 is isolated from a human organism. These are bacilli. The size of cells of a one-day agar culture is (5 - 10) x (0.7 - 1.2 µm). They grow as a chain of 3 - 5 cells. Mobile, gram-positive. Spores located subterminally, do not form capsules. Colonies on a meat-peptone agar are slightly protruding, yellowish, 2 - 4 mm in diameter. On a meat-peptone broth they grow as a film on the surface and as flakes on the bottom. They grow in a 7% NaCl, under aerobic and anaerobic conditions at a temperature of from 25 to 40°C, optimum 36 - 37°C. Do not ferment lactose, glucose, saccharose, mannitol, arabinose, xylose. They evolve hydrogen sulphide, do not form indole. Do not coagulate plasma, do not give a positive dermonecrotic test response.

The strain is sensitive to erythromycin, oleandomycin, methycillin, carbenicillin, restomycin, levomycetin; resistant to polymixin, linkomycin, monomycin.

The strain produces an antibacterial substance of a wide spectrum of action.

The strain liberates into the ambient medium proteolytic enzymes decompsing albumin, fibrin, hemoglobin. It produces immunomodulating substances lowering allergization.

The strain is non-toxic and non-pathogenic.

The novel strain Bacillus pulvifaciens 535 is isolated from a non-purulent wound of a patient. This is a bacillus, the size of cells of a one-day agar culture is $(2 - 4) \times (0.6 - 0.8)$ $\mu$m. Bacteria are virulent, when grown on a meat-peptone agar they form a wrinkly film. Form spores, do not form capsules. Gram-positive. Colonies on a meat-peptone agar are rough, with scalloped edges, a slight pink shade, 2 - 9 mm in diameter, they grow both under aerobic and anaerobic conditions. They are multiplied at a temperature of 15 - 50$^{o}$C, optimum - 36 - 37$^{o}$C. Cleave glucose, saccharose, maltose, dulcite, galactose to an acid without evolution of a gas. Do not ferment lactose, mannitol, xylose, ramnose, lysine, arginine, ornitine. Do not form hydrogen sulphide and indole. Do peptonize milk. Do not produce lecitinase. Evolve acetylmethylcarbinol and catalase, do not hydrolyze starch.

The strain is sensitive to erythromycin, ampicillin, methycillin, benzylpenicillin, oxacillin, linkomycin, neomycin, levomycetin, monomycin, ristomycin, kanamycin, tetracyline, oleandomycin; insensitive to polymixin.

The strain _Bacillus pulvifaciens_ produces proteolytic enzymes decomposing albumin, hemoglobin, fibrin, an antibiotic of a wide spectrum of action and an immunomodulator. Allegedly pathogenic microorganisms sensitive to the antibiotic are:

staphylococci, streptococci, colibacilli, blue pus bacilli, proteus, salmonella, dysentery bacilli, clebsiella, yeast fungi. It does not inhibit the growth of a saprophylous microflora. Furthermore, the antibiotic is fully destroyed rather rapidly (during several hours) which excludes origination of a food allergy. Proteolytic enzymes contribute to assimilation of fodders.

Principal distinctions of this strain from <u>Bacillus subtilis</u> 534 - lack of hydrolysis of starch and cleavage of mannitol.

The strain is non-toxic and non-pathogenic.

The non-harmful character of the agent according to the present invention, its ability of penetration from the gastro-intestinal tract, to get concentrated and preserved for a period of from 2 hours to 14 days in tissues of the inflammatory focus, spleen, lympth nodes, lymphoid follicles of the intestine and liver have been established in experiments on animals and justified in clinic on human beings.

In the determination of an acute toxicity of the agent according to the present invention use was made of three series of the preparation obtained from a live culture of the strain <u>Bacillus subtilis</u> 534. Each series was administered in a single dose intraperitoneally to 3 white mice and 3 white rats of the Vistar line respectively in the doses of 10 and 20 bln, cells in 1 $cm^3$ of a 0.9% solution of sodium chloride. All the animals survived. They had a good appetite. The animals were slaughtered on the 3-rd, 7-th and 14-th day of the experiment. Histological studies revealed no inflammatory or dystrophic changes in the brains, lungs, kidneys, liver, heart were found. In the spleen there was noted an increase in the size of follicles and the number of lymphohistocytic units in the red pulp.

For the study of the degree of an acute toxicity three series of the preparation from the strain Bacillus subtilis 534 were administered each to 6 white mice and 6 rats of the Vistar line in a single portion with meals in the doses of 10 and 20 bln. cells respectively. All the animals survived. They were slaughtered on the next day. Histological studies of the brains, myocardium, lungs, kidneys, stomach, small and large intestine revealed no changes relative to the animals of the control group (10 mice and 10 rats). In the liver there was noted a certain increase in the number of lymphohistocytic infiltrates along the way of portal tracts. In the spleen an increased size of follicles, the appearance of a great number of lymphohistocytic units in the red pulp and giant polynuclear cells were noted.

The strains Bacillus pulvifaciens 535 and Bacillus sp. 538 preliminarily killed by formalin vapours were administered intraperitoneally to 20 white mice in the dose of 2 bln. cells in 1 $cm^3$ of a 0.9% solution of sodium chloride. Live cultures of the same strains in the doses of 10 and 20 bln. cells were administered to other 30 white mice and 30 white rats of the Vistar line. All the animals survived. They had a good appetite. The animals were slaughtered on the 3-rd, 7-th and 14-th day of the experiment. In histological studies no inflammatory or dystrophic changes were found in the brains, lungs, kidneys, heart, liver. In the spleen there was noted an increase in the size of follicles and number of lymphohistocytic units in the red pulp.

To 15 white mice and 15 white rats of the Vistar line 10 and 20 bln. cells of a suspension of a live lyophilized culture of the strain Bacillus pulvifaciens 535 were administered per os. The animals survived. They were slaughtered on the next day. Histological studies of the brains, myocardium, lungs, kidneys, stomach, small and large intestine revealed no changes as compared to the animals of the control group. In the liver there was noted an increase in the size of follicles, appearance of a great number of lymphohistocytic units in the red pulp and of giant polynuclear cells.

To 12 rabbits live one-day cultures of the strains Bacillus pulvifaciens 535 and Bacillus sp, 538 were hypodermally administered to each in the doses of 100,000; 1 mln., 10 mln, 1 bln. and 5 bln. cells in 1 cm$^3$ of a 0.9% solution of sodium chloride. The general conditions of the animals did not change, all of them survived. No local changes were noted in the spot of administration, but spore-bearing bacteria were inoculated from the infection zone for 2 weeks.

In the determination of a chronical toxicity three series of the preparation based on the strain Bacillus subtilis 534 were administered each in 3 white mice and 3 white rats of the Vistar line respectively in the doses of 200 mln. and 1 bln. of cells in 1 cm$^3$ of a 0.9% solution of sodium chloride intraperitoneally for 30 days. Furthermore, each of the three series of the preparation was administered per os to 6 white mice and 6 white rats of the Vistar line in the same doses respectively. All the animals survived. The mass of the animals of the test groups as compared to the control ones (10 mice and 10 rats) was by 11 - 17% higher (p < 0.05). The mice and rats of the test groups had bright white and fluffy hair. The animals were slaughtered on the 31-st day. Histological studies of the brains, myocardium, kidneys, lungs, stomach, small and large intestine revealed no changes. In the liver there were noted greater amounts, as compared to the animals of the control groups, of lymphohistocytic infiltrates located along the way of portal tracts. In the spleen of the animals of the test groups there were noted lymphoidization of the red pulp and increase in the size of follicles, though no giant polynuclear cells were detected.

In the course of the experiments on studying chronical toxicity 6 white mice gave birth on the 11-21-st day to 23 youngsters without malformations. Later on they also gave birth to 7 youngsters without malformations.

In the determination of a chronical toxicity live one-day cultures of the strains Bacillus pulvifaciens 535 and Bacillus sp. 538 were daily administered for 30 days intraperitoneally to 40 white mice in a dose of 200 - 500 mln. cells and to 30 rats of the Vistar line in the dose of 2 bln. cells in 1 $cm^3$ of a 0.9% solution of sodium chloride. Furthermore, to 48 white mice and 30 rats of the Vistar line daily for 30 days 200 mln. cells and 1 bln. cells of a live lyophilized culture of the above-mentioned strains respectively were administered per os with water. All the animals survived. The mass of the animals of the test group was by 11 - 19% higher than that of the animals of the control group. The mice and rats of the test group had bright white and fluffy hair. The animals were slaughtered. Histological studies of the brains, myocardium, lungs, kidneys, stomach, small and large intestine revealed no changes. In the liver there were noted greater numbers, as compared to the animals of the control group, of lymphohistocytic infiltrate located both along the way of portal tracts and outside them. In the spleen of the animals of the test group there were observed the lymphoidization of the red pulp and an increase in the size of follicles, though no giant polynuclear cells were detected.

In the course of the experiments on studying of a chronical toxicity 9 white mice gave birth, on the 11 - 21-st day, to 54 youngsters without malformation. The latter gave birth to 9 mice without malformations. Therefore, live cultures of the strains even upon an intraperitoneal administration do no effect the development of a fetus and do not cause genetic disturbances.

Three series of the preparation based on the strain Bacillus subtilis 534 were administered each in the dose of 2 bln. cells in 1 $cm^3$ of a 0.9% solution of sodium chloride 6 times in a day hypodermally to 4 guinea pigs. On the 31-st day the preparation was injected intradermally to all the guinea pigs in the dose of 2 bln. cells in 1 $cm^3$ of a 0.9% solution of sodium chloride. No local or general symptoms of the allergic response were noticed.

Live cultures of the strains Bacillus pulvifaciens 535 and Bacillus sp. 538 in the dose of 2 bln. cells of each in 1 $cm^3$ of a 0.9% solution of sodium chloride were administered 6 times a day hypodermally to 24 guinea pigs (1 strain to 12 animals). On the 31-st day to all the guinea pigs intradermal injections were made of 2 bln. cells of microorganisms of the same strains in 1 $cm^3$ of a 0.9% solution of sodium chloride. No local or general signs of the allergic response were noted.

For the determination of stability of the preparation of the present invention, 9 capsules of the preparation made on the basis of the strains Bacillus subtilis 534, Bacillus sp. 538, Bacillus pulvifaciens 535 in tightly closed flasks were stored at room temperature for 7 years. Additional 9 capsules were stored for 3 months at the temperature of -20 to $-22^{o}C$, 9 capsules were stored for 2 months at the temperature of $110^{o}C$. The number of live bacteria in the capsules was determined by the method of inoculation of serial dilutions.

Prior to the beginning of the experiments the content of bacteria in a capsule was equal to 5.4 ± 0.5 bln. cells, upon storage for 7 years it was 5.2 ± 0.5 bln. cells, upon storage at a temperature of -20 to $-22^{o}C$ - 5.4±0.6 bln.cells, upon storage at the temperature of $110^{o}$ C - 5.3±0.6 bln.cells. Therefore, the obtained data demonstrate that the agent according to the present invention can be stored for up to 7 years within a broad temperature range.

The strains Bacillus subtilis 534, Bacillus sp.538 Bacillus pulvifaciens 535, E.coli M17, Lb.plantarum 8-PA-3, B.bifidum N 1 were grown by way of 5-fold reinoculations on media containing radionuclides $^{14}C$, $^{131}J$, $^{99}Tc$. Afterwards, each of the strains in the dose of 2 bln. cells in 1 $cm^3$ of a 0.9% solution of sodium chloride was administered per os to 5 mice. All the animals survived, no signs of intoxication were observed. Consequently, repeated inoculations have shown that the agent according to the present invention is stable: virulent and pathogenic properties of bacteria were not revealed.

For a better understanding of the present invention, some specific examples illustrating preparation of the agent according to the present invention and testing thereof are given hereinbelow.

Example 1

The strain Bacillus subtilis 534 is isolated from a non-purulent wound of a patient. It has proven to be capable of penetrating from the gastro-intestinal tract into the tissues of the internal medium without causing unfavourable negative effects. The strain is grown for one day at the temperature of $37^{o}C$ on a 1.5% peptone agar with $H_3$-leucine tagged with $^{14}C$. The strain is rinsed with a 0.9% solutions of sodium chloride and lyophilized.

The agent according to the present invention is thus obtained which contains 500 mln. live cells of the above-specified strain in 1 mg of dry substance. The activity of 1 mg of the agent according to the present invention is 0.1 MBq.

The agent according to the present invention is then subjected to testing. A suspension of the agent is prepared in a 0.9% solution of sodium chloride. Each of 4 healthy rabbits and 4 rabbits with wounds in the back area is administered with 10 $cm^3$ of the suspension containing 1 bln. live cells of bacteria of the above-mentioned strain (2 mg of dry solides).

Radiodiagnosis after 24 hours shows the following distribution of the radionuclides in the healthy rabbits: in the spleen - 23-36% of the administered dose, in lymph nodes of the peritoneal cavity - 7-12%, in lymphoid follicles of the intestine - 3-5%, liver - 4-9%. In the wound rabbits (with an inflammatory focus) the distribution of the radionuclides was the following: in the wound area - 29-41%, in the spleen - 12-16%, lympth nodes of the peritoneal cavity - 2-4%, lymphoid follicles of the intestine - 1-2%, liver - 7-11%. After 72 hours the number of radionuclides in these organs and tissues decreased by 50-82% and they were fully withdrawn by the 7-th day.

Example 2

Into a suspension containing 10 bln. cells of bacteria of the strain Bacillus pulvifaciens 538 in a 0.9% solution of sodium chloride $^{99}$Tc with the activity of 800 MBq is introduced. The mixture is subjected to incubation in a thermostat at the temperature of 37°C for 6 hours, whereafter it is centrifugated. The supernatant liquid is removed. The resulting agent according to the present invention contains about 500 mln. live cells of bacteria in 1 mg with the radionuclide $^{99}$Tc. The activity of 1 mg of the agent is about 10 MBq. A patient is orally administered with 5 mg of the agent of this invention with the 99$^{T}$c radionuclide. Two hours thereafter a scanogram of a healthy spleen is obtained.

Example 3

The strain Bacillus pulvifaciens 535 is grown on a 2% peptone broth with albumin tagged with $^{131}$J for 12 hours at the temperature of 37°C. The resulting culture mixture is centrifugated, the residue is separated and dried to give the agent according to the present invention containing about 100 mln. live cells in 1 mg with the radionuclide $^{131}$J. The activity of 1 mg of the agent

- 14 -

according to the present invention is O.01 MBq. The agent in the dose of 1 g is orally administered to rabbits with an experimental abscess of a limb, pneumonia and paranephritis. A scanogram of the inflammatoty foci in all of the rabbits was obtained.

Example 4

The strain Lb. plantarum 8-PA-3 is grown on a milk with $H_3$ leucine tagged with $^{14}C$. The resulting culture mixture is dried to give the agent according to the present invention containing about 100 mln. live cells of bacteria in 1 mg with the radionuclide $^{14}C$. The activity of 1 mg of the agent according to the present invention is 0.1 MBq.

The agent in the dose of 25 mg is twice a week administered to 5 hens with leukosis. The average lifespan of the hens was 123 days, whereas in 5 ill hens of the control group it was 71 days.

Example 5

The strain B.bifidum N 1 is grown on a Blaurock nutrient medium for 7 days at the temperature of $37^{\circ}C$ under anaerobic conditions and then lyophilized. The resulting agent according to the present invention contains 100,000 live cells of bacteria in 1 mg with the radionuclide $^{14}C$. The activity of 1 mg of the agent of this invention is 0.01 MBq.

A suspension of the thus-prepared agent in a 0.9% solution of sodium chloride is then obtained. 0.2 $cm^3$ of the suspension containing 50-500 live cells of bacteria is administered to each of 5 white mice. Historadiographic studies of the radiotag of the bacteria in the spleen tissues have shown their presence there within 6-48 hours.

Example 6

The strain E.coli M17 is grown on an Endo medium for 24 hours at the temperature of $37^{\circ}C$. The resulting culture is rinsed-off with a 5% solution of glucose and lyophilized at a temperature drop from -40 to $+40^{\circ}C$ under a pressure of from 5 to 70 Pa for 24 hours. The resulting agent contains $2 \times 10^9$ live cells of bacteria in 1 mg. The agent incorporates a radionuclide introduced as described in Example 2 hereinbefore. The activity of 1 mg of the resulting preparation is 0.2 MBq. The rabbit was administered per os with 10 mg of the preparation. A scanogram of the spleen was obtained 3 hours after administration of the preparation. The control investigation showed a full withdrawal of the radionuclides from the organism within one day.

## Industrial Applicability

The agent according to the present invention is useful in veterinry  and medicine for transportation of radionuclides and carrying out radiological investigation of inflammatory foci of different localization, as well as spleen, lymph nodes, lymphoid follicles of the intestine. The agent of this invention with radionuclides incorporated therein can be useful as a pharmaceutical preparation for the treatment of leukoses and diseases of the spleen, lymph nodes of a tumoral nature.

The agent of the present invention can be also used for the provision of novel pharmacological preparations as a base enabling transportation of the active principle from the gastro-intestinal tract to the tissues of inflammatory foci, spleen, lymph nodes, liver.

- 16 -

C L A I M S:

1. An agent for transportation of radionuclides in a live organism, characterized in that it comprises cells of at least one non-pathogenic organism capable of penetrating from the gastro-intestinal tract into tissues of the spleen, lymph nodes, lymphoid follicles of the intestine and into an inflammatory focus and concentrating therein; and in that it contains 10 to $2 \times 10^9$ live cells of the microorganisms in 1 mg of the agent.

2. An agent according to claim 1, characterized in that as the bacteria it incorporates the strain Baccilus subtilis 534 deposited on March 28, 1988 at the All-Union Collection of microorganisms of the Institute of biochemistry and physiology of microorganisms, the USSR Academy of Sciences, and registered under No. B-1666D.

3. An agent according to Claim 1, characterized in that as the bacteria it concorporates the strain Bacillus sp. 538 deposited on June 20, 1988 at the All--Union Collection of industrial microorganisms of the All-Union Research Institute of genetics and selection of industrial microorganisms and registered under No. BKPM B-4401.

4. An agent according to Claim 1, characterized in that as the bacteria it incorporates the strain Bacillus pulvifaciens 535 deposited on April 14, 1988 at the All-Union Research Institute of genetics and selection of industrial microorganisms and registered under No. BKPM B-4348.

5. An agent according to Claim 1, characterized in that as the bacterial it incorporates the strain Escherichia coli M17.

6. An agent according to Claim 1, characterized in that as the bacteria it incorporates the strain Lactobacillus plantarum 8-PA-3.

- 17 -

7. An agent according to Claim 1, characterized in that as the bacteria it incorporates the strain Bacteriumbifidum bifidum N 1.

# INTERNATIONAL SEARCH REPORT

## I. CLASSIFICATION OF SUBJECT MATTER (if several classification symbols apply, indicate all) 6

According to International Patent Classification (IPC) or to both National Classification and IPC

IPC$^4$ – A 61 K 43/00

## II. FIELDS SEARCHED

| Minimum Documentation Searched 7 | |
|---|---|
| Classification System | Classification Symbols |
| IPC$^4$ | A 61 K 43/00,39/00, 49/02, C 12 N 1/00, 1/20 |

| Documentation Searched other than Minimum Documentation to the Extent that such Documents are Included in the Fields Searched 8 |
|---|
| |

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| A,P | EP,A1,0277930,(UNIVERSITE LIBRE DE BRUXELLES) 10 August 1988 (10.08.88),see the claims | 1-7 |
| A | Referativny zhurnal "Biologia" 041. Mikrobiologia obschaya, No.5, (Moscow,VINITI,SSSR),Reid Graeme "Pulse labeling of yeast cells and spheroplasts", see page 5,abstract 5L46,("Meth. Enzymol.Vol.97. Pt K.;" New York e.a.,1983, 324-329 (Engl.) | 1-7 |
| A | GB,A,2042887,. (NEW ENGLAND NUCLEAR CORPORATION), 01 October 1980 (01.10.80), see the claims | 1-7 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 28 April 1989 (28.04.89) | 23 May 1989 (23.05.89) |
| International Searching Authority | Signature of Authorized Officer |
| ISA/SU | |